# EUROPEAN PATENT APPLICATION

(11) **EP 1 767 147 A1**
(43) Date of publication of application: **28.03.2007**
(21) Application number: 05752967.9
(22) Date of filing: 21.06.2005
(51) Int. Cl.: A61B 5/0478

(54) **BRAIN WAVE DETECTION ELECTRODE DEVICE AND PACKAGE**

(30) Priority: 25.06.2004 JP 2004188937; 23.07.2004 JP 2004216013
(71) Applicant: Olympus Corporation, Tokyo 151-0072 (JP); Brain Functions Laboratory, Inc., Kawasaki-shi, Kanagawa 2100855 (JP)
(72) Inventor: KUMADA, Yoshiyuki, Suginami-ku, Tokyo 1670053 (JP); MUSHA, Toshimitsu c/o Brain Functions Laboratory,, Kawasaki-ku, Kanagawa 2100855 (JP)
(74) Representative: von Hellfeld, Axel
(86) International application number: PCT/JP2005/011359
(87) International publication number: WO 2006/001276

(57) **Abstract**

The labor involved in cleaning for each examination is reduced, the examination can be carried out hygienically, and the convenience of use is improved. An electrode apparatus for detecting brain waves which is arranged in contact with the scalp and which detects brain wave signals is provided. The electrode apparatus for detecting brain waves comprises a rod-shaped electrode apparatus main body having an electrode disposed at the tip thereof, a cap which is mountable on the tip of the electrode apparatus main body and which has an elastic member which contains an electrolyte and which is disposed making close contact so as to cover the electrode, and a connection means which detachably connects the cap to the electrode apparatus main body.

## Description

### [Technical Field]

The present invention relates to an electrode apparatus for detecting brain waves and package.

### [Background Art]

Conventionally, with the goal of the early detection of Alzheimer's disease, analysis of the functional status of brain has been performed and various analytical methods of the functional status of brain have been proposed (for example, refer to Non-patent Document 1 and Non-patent Document 2). In these analytical methods brain wave signals are detected and judgments are made based on the smoothness of the spatial transmission of the brain wave signals and the standard deviation thereof. When the activity of the brain nerve cells is normal, the brain wave signals are transmitted smoothly and the variation therein is small, but when Alzheimer's disease is progressing and the activity of brain nerve cells has deteriorated, there is a tendency for the smoothness of the spatial transmission of brain wave signals to be lost and for the standard deviation thereof to increase.

In order to carry out this type of analysis with good accuracy, it is essential for the brain wave signals to be detected with good accuracy. Brain wave signals are detected by bringing a plurality of electrodes into contact with the scalp, but due to the presence of hair and lack of uniformity in the shape of the head, it is common for the electrodes to be brought into contact with the scalp by means of a paste in order to realize a reliable state of contact between the electrodes and the scalp.

However, paste-less detection methods for brain wave signals, in which a paste is not used, have also been proposed (for example, refer to Patent Document 1 and Patent Document 2). These detection methods are methods in which a fibrous piece into which an electroconductive solution has been absorbed or a foam material impregnated with a hydrated gel which provides conductivity are brought into contact with the scalp as electrodes, respectively.

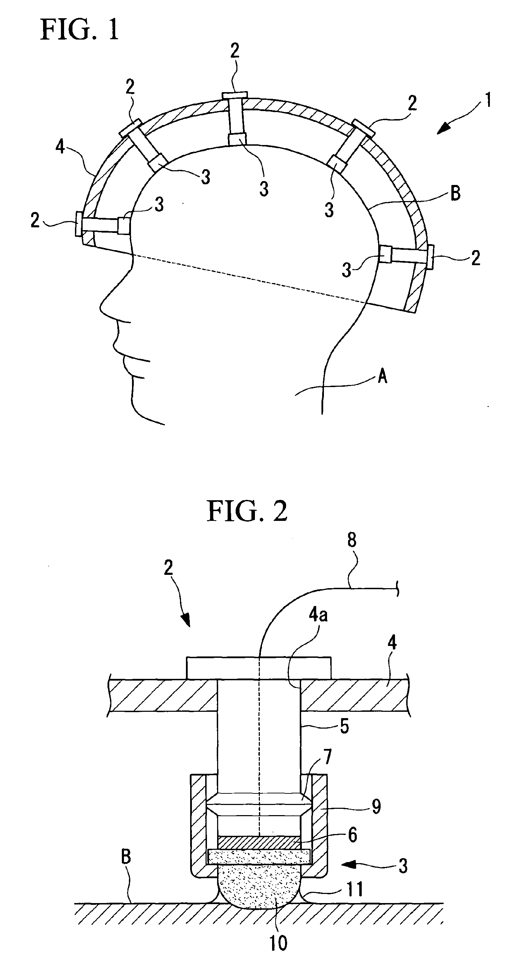

[Non-Patent Document 1] Brain Functions Laboratory, Inc., "DIMENSION New brain wave analysis method for detecting neuronal dysfunction early in Alzheimer's disease", online, Search Date: March 8, 2004; Internet URL: http://www.bfl.co.jp/abst-32.html
[Non-Patent Document 2] Brain Functions Laboratory, Inc., "DIMENSION Diagnosis method of neuronal dysfunction, Online, Search Date: March 8, 2004; Internet URL: http://www.bfl.co.jp/abst-31.html
[Patent Document 1] Japanese Unexamined Patent Application, First Publication No. Hei 10-165386 (page 2, and others)
[Patent Document 2] Japanese Unexamined Utility Model Application, First Publication No. Hei 2-63811 (FIG. 1, and others)

### [Disclosure of Invention]

It is necessary for the detection of brain waves to be carried out by correctly arranging a plurality of electrodes, and it is common for the electrodes to be fixed in position in a designated holder which is put onto the head of the subject being examined. In this case, to position the electrodes in a state of contact with the skin, it is necessary for the tips of the electrodes to be cleaned for each examination.

The present invention was achieved in consideration of the above-mentioned circumstances, and the present invention has objects of providing an electrode apparatus for detecting brain waves and a package with which it is possible to reduce the labor involved in cleaning for each examination and to carry out examinations hygienically.

In order to achieve the above-mentioned objects, the present invention provides the following means.
The present invention provides an electrode apparatus for detecting brain waves which is disposed in contact with the scalp and detects brain wave signals, comprising a rod-shaped electrode apparatus main body having an electrode disposed in a tip thereof, a cap which is mountable on the tip of the electrode apparatus main body and which has an elastic member containing an electrolyte and disposed in close contact so as to cover the electrode, and a connection means for connecting the cap in a detachable manner to the electrode apparatus main body.

According to the present invention, by putting a cap on the rod-shaped electrode apparatus main body, which has an electrode disposed at the tip thereof, the electrode is covered, and an elastic member which contains an electrolyte and which is provided in the cap is disposed in close contact on the electrode. As a result, when the elastic member of the tip of the cap is brought into contact with the scalp, the electrolyte which is contained in the elastic member wets the scalp, the electrode and the scalp become electrically connected, and the brain wave signals are detected by means of the electrode.
In this situation, due to the action of the connection means, the cap is removably mounted on the electrode apparatus main body. Therefore, by means of the action of the connection means during use, the cap is mounted on the tip of the electrode apparatus main body and covers the electrode, and when not in use, the cap can be removed from the tip of the electrode apparatus main body. In other words, it is possible to make the cap a disposable part. As a result, simply by replacing the cap and without replacing the electrode apparatus main body, it is possible to easily carry out the procedure for detecting brain waves with respect to many subjects, hygienically and at low cost.

In addition, the present invention provides an electrode apparatus for detecting brain waves which is disposed in contact with the scalp and detects brain wave signals, comprising a rod-shaped electrode apparatus main body having a first contact point disposed in a tip thereof, a cap mounted such that the tip of the electrode apparatus main body is covered and having a second contact point for contact with the first contact point, and a connection means for connecting the cap in a detachable manner to the electrode apparatus main body, the cap having an electrode for connection to the second contact point and an elastic member containing an electrolyte and disposed in close contact so as to cover the electrode.

According to the present invention, by the action of the connection means during use, when the cap is mounted on the tip of the electrode apparatus main body, the electrode apparatus main body and the electrode of the cap are electrically connected by the connection of the first contact point and the second contact point. Since an elastic member containing an electrolyte is provided in the cap, when the tip of the cap is brought into contact with the scalp, in addition to the elastic member being elastically deformed and making close contact with the scalp, the electrolyte contained in the elastic member wets the scalp and the electrode and the scalp become electrically connected. As a result, it is possible for brain wave signals from the scalp to be detected by the electrode.

In the above-described invention, it is preferable for the connection means to have a seal member which maintains a contact section of the first contact point and the second contact point in a watertight condition, when the cap is mounted on the electrode apparatus main body.
When the cap is mounted on the electrode apparatus main body, since the connection means maintains the contact section of the first contact point and the second contact point in a watertight condition by the action of the seal member, the penetration of moisture, dust and dirt into the contact section of the contact points is prevented, and thereby it is possible to prevent the occurrence of problems such as poor contact before they develop.

In addition, the present invention provides an apparatus for detecting brain waves in which a plurality of cap sections is integrally connected by means of a linking section.

According to the present invention, by putting a plurality of caps sections integrally connected by means of a linking section on the tips of a plurality of electrode apparatus main bodies provided in the apparatus for detecting brain waves, it is possible to easily arrange a plurality of electrode apparatuses for detecting brain waves together. Since the arrangement of the caps and the electrode apparatuses for detecting brain waves is almost constant, by integrating a plurality of cap sections by means of the linking section, it is possible to reduce the number of parts and to simplify management.

In the above-mentioned invention, it is preferable for the linking section to comprise a flexible material.
By means of the linking section comprising a flexible material, it is possible for the cap sections to be mounted by deforming the linking section to match the positions of all of the electrode apparatus main bodies of the plurality of electrode apparatuses for detecting brain waves whose positions have been adjusted to match the shape of the head of the subject.

In the above-mentioned invention, an indicator mark indicating the direction for mounting may be provided on the linking section.
When a plurality of engaging cylindrical parts are integrally formed, when they are being put onto the plurality of electrode apparatuses for detecting brain waves, it is convenient for the direction therefor to be indicated. In other words, since the predetermined spacing between the electrode apparatuses for detecting brain waves is not uniform, it is possible to make the mounting operation simple by indicating the mounting direction using an indicator mark.

In addition, in the above-mentioned invention, the connection means may also comprise an elastic supporting member which is fixed to either one of the cap and the electrode apparatus main body in an engaging section of the cap and the electrode apparatus main body, and which maintains a connected condition by means of frictional force with the other one of the cap and the electrode apparatus main body.
When the cap is engaged with the electrode apparatus main body, the elastic supporting member, which is provided on either one of the cap and the electrode apparatus main body, is elastically deformed, a frictional force is generated between it and the other of the cap and the electrode apparatus main body, and the connected state is maintained. As a result, it is possible to easily mount and remove the cap from the electrode apparatus main body.

In addition, in the above-mentioned invention, the elastic supporting member may comprise an O-ring or Y-packing.
By the elastic supporting member comprising an O-ring or Y-packing, it is possible to block and tightly seal the entirety of the engaging section of the cap and the electrode apparatus main body, it is possible to maintain the cap in a connected condition on the electrode apparatus main body by frictional force, and it is possible to prevent the penetration of dust, dirt, and moisture into the connection section.

In addition, in the above-mentioned invention, the connection means may comprise a female screw thread provided on one of the cap and the electrode apparatus main body, and a male screw thread provided on the other one of the cap and the electrode apparatus main body and which engages the female screw thread.
In addition, in the above-mentioned invention, the connection means may comprise a projection which projects in a direction orthogonal to the connection direction and which is provided in one of the cap and the electrode apparatus main body, and a groove which receives the projection when in a connected condition and which is provided on the other one of the cap and the electrode apparatus main body.
By means of engaging or disengaging the female and male screw threads, the cap can be easily mounted or removed from the electrode apparatus main body, and it is possible to maintain a reliable engaged condition. In addition, by means of the engagement of the projection and groove, easy and reliable mounting and removal is possible.

In the above-mentioned invention, an area of a surface of the tip of the cap which makes contact with the scalp is not less than approximately 19 mm² and not greater than approximately 314 mm².

According to the present invention, when the electrode apparatus for detecting brain waves is pressed against a scalp having hair, the surface of the tip of the cap having an elastic member containing an electrolyte makes contact with the scalp by pushing the hair aside. Some of the hair of the head is sandwiched between the surface of the tip of the cap and the scalp, but by setting the area of the surface of the tip of the cap to approximately 19 mm² or greater, it is possible for the hair to be parted or pushed aside and for contact to be made with a sufficient area of the exposed scalp, and it is possible to reduce contact resistance. In addition, the amount of hair that is sandwiched between the surface of the tip of the cap and the scalp increases as the area of the surface of the tip of the cap increases. Therefore, increasing the area of the surface of the tip of the cap does not lead to improvements in contact resistance. In fact, when the area is set at approximately 314 mm² or greater, there is a likelihood of interference with neighboring electrodes, and therefore, this is not preferable. When the size of the area is set to be 314 mm² or less, such problems do not arise.

In the above-mentioned invention, it is preferable for the electrode to comprise metal or to be metal plated.
By the metal electrode comprising metal or being metal plated, it is possible to reduce electrical resistance and it is possible due to anti-corrosion effects to prevent increases in electrical resistance which accompany use.

In addition, in the above-mentioned invention, the electrolyte may be a physiological saline solution. In addition, the electrolyte may be a mixture of a physiological saline solution and alcohol. Due to these electrolytes, the electrical resistance is low, and there is no degradation in the detected brain wave signal. In addition, by mixing in alcohol, there is a cleaning effect with respect to the sebum of the scalp, and it is possible to further reduce contact resistance.

In addition, in the above-mentioned invention, the electrode apparatus main body may comprise a plurality of electrodes, and a total area of surfaces of the tips of the caps which are disposed making close contact so as to cover all the electrodes is not less than approximately 19 mm² and not greater than approximately 314 mm².
By ensuring that the total area is not less than approximately 19 mm², it is possible to reduce contact resistance. In addition, by division into a plurality of tip sections, the hair which is pushed aside is disposed between the tips, and it is possible to bring the tip sections into close contact with the scalp.

In addition, the present invention may comprise an electrode having the shape of comb teeth in the electrode apparatus main body, and the total area of the surface of the tip of the cap which is disposed in close contact so as to cover the electrode is not less than approximately 19 mm² and not greater than approximately 314 mm².
The plurality of tip sections of the integrated metal electrode function like the teeth of a comb, and it is possible for the hair to be pushed aside and for contact to be made with a sufficient area of the exposed scalp, and it is possible to reduce contact resistance.

In addition, in the above-mentioned invention, the tip section of the electrode may be formed having an approximately spherical surface shape, and the surface of the tip of the cap may be formed having an approximately spherical surface shape matching the spherical surface section of the electrode.
By doing this, even when the shape of the head is complicated and it is not possible to bring the metal electrode into perpendicular contact with the scalp, it is still possible to reduce contact resistance and to achieve reliable contact.

In addition, the present invention provides a package comprising the above-described cap and a packing member for housing the cap in an airtight state.
According to the present invention, by housing the cap in an airtight state by means of the packing member, the volatilization of the electrolyte contained in the elastic member is prevented, and provision in an immediately usable condition is possible.

In the above-mentioned invention, it is preferable for the invention to further comprise a cover member for covering the surface of the elastic member in the cap.
As a result of this, by being housed in the packing member in a condition in which a cover covers the surface of the elastic member, it is possible to prevent in advance the occurrence of any problems such the spilling of the electrolyte from the elastic member within the packing member.

In addition, in the above-mentioned invention, a humidity sensor that is visible from the outside may be disposed within the packing member.
By doing this, it is possible to indicate the humidity inside the packing member by the action of the humidity sensor. When the electrolyte of the elastic member has dried out, it is not possible to accurately detect brain wave signals using that cap. Therefore, by indicating the point that the cap cannot be used in advance by means of the humidity sensor, it is possible to prevent unnecessary mounting procedures and unnecessary examination operations.

In addition, in the above-mentioned invention, the above-mentioned packing member may house a plurality of caps.
By housing a plurality of caps which are to be used at the same time in the same packing member, it is possible to reduce the number of packing members and to reduced costs, and to make the management of humidity easy.

In addition, in the present invention, it is preferable for the plurality of caps to be housed in the packing member corresponding to their arrangement at the time of the brain wave detection.
Since a plurality of electrodes are used at the same time in brain wave detection, by housing the plurality of caps which are to be used at the same time in a manner corresponding to their arrangement at the time of the brain wave detection, it is possible to prevent reuse due to failure to replace caps and to prevent oversights in the mounting of caps.

In addition, in the above-mentioned invention, it is preferable for an identical identifying code to be provided on the cap and on the packing member.
By providing the same identifying code on caps which will be used at the same time, it is possible to prevent oversights in the replacement of caps.

### [Brief Description of Drawings]

[FIG. 1] is a schematic diagram showing a brain wave examination apparatus according to a first embodiment of the present invention in a condition mounted on the head of a subject.
[FIG. 2] is a longitudinal cross-section showing the first embodiment of an electrode apparatus for detecting brain waves used in the brain wave examination apparatus of FIG. 1.
[FIG. 3] is a longitudinal cross-section showing a condition in which a cap has been removed from an electrode apparatus main body of the electrode apparatus for detecting brain waves of FIG. 2.
[FIG. 4] is a partial longitudinal cross-section showing a variant of the cap of the electrode apparatus for detecting brain waves of FIG. 2.
[FIG. 5] is a longitudinal cross-section showing a second embodiment of the electrode apparatus for detecting brain waves used in the brain wave examination apparatus of FIG. 1.
[FIG. 6] is a longitudinal cross-section of showing a variant of the electrode apparatus for detecting brain waves of FIG. 5.
[FIG. 7] is a longitudinal cross-section showing a third embodiment of the electrode apparatus for detecting brain waves used in the brain wave examination apparatus of FIG. 1.
[FIG. 8] is a planar view showing caps for mounting in the electrode apparatus for detecting brain waves of FIG. 7.
[FIG. 9] is a diagram showing a cap package according to the first embodiment of the present invention.
[FIG. 10] is a longitudinal cross-section showing the case in which the cap of the electrode apparatus for detecting brain waves shown in FIG. 5 is used as the cap housed inside.
[FIG. 11] (a) and (b) are a perspective view (a) showing one example of a packing member in which a plurality of caps can be stored, and a lateral view (b) of a cap.
[FIG. 12] is a longitudinal cross-section showing the case in which caps not having an electrode of the electrode apparatus for detecting brain waves shown in FIG. 7 are used as the caps housed inside.
[FIG. 13] is a longitudinal cross-section showing the case in which a cap having an electrode is used as the cap housed inside.
[FIG. 14] is a perspective diagram showing the situation in which the caps of FIG. 12 are housed in the packing member.
[FIG. 15] is an overall constitutional diagram showing a brain function analysis system equipped with the electrode apparatus for detecting brain waves according to a fourth embodiment of the present invention.
[FIG. 16] (a) to (c) are a lateral view (a), a front view (b), and a planar view (c) of a human head showing the arrangement of the electrode apparatus for detecting brain waves of FIG. 15.
[FIG. 17] is a perspective view of the disassembled tip section of the electrode apparatus for detecting brain waves of FIG. 15.
[FIG. 18] is a longitudinal cross-section showing the situation in which the electrode apparatus for detecting brain waves of FIG. 17 is in contact with the scalp.
[FIG. 19] is a graph showing the relationship between the average value of the contact resistance and the external dimensions of the tip section of the electrode apparatus for detecting brain waves of FIG. 15.
[FIG. 20] is a perspective diagram showing the tip section of the electrode apparatus for detecting brain waves according to a fifth embodiment of the present invention.
[FIG. 21] is a longitudinal cross-section showing a variant of the electrode apparatus for detecting brain waves of FIG. 20.
[FIG. 22] is a perspective diagram showing the electrode apparatus for detecting brain waves of FIG. 21.

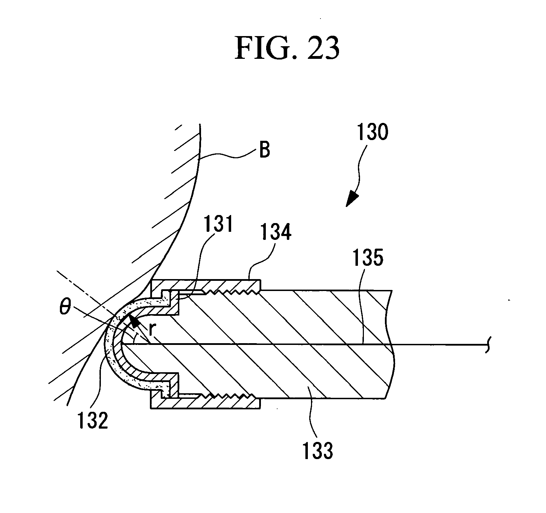

[FIG. 23] is a longitudinal cross-section showing the tip section of an electrode apparatus for detecting brain waves according to a sixth embodiment of the present invention.

### [Best Mode for Carrying Out the Invention]

A brain wave examination apparatus 1, an electrode apparatus for detecting brain waves 2, and a cap 3 according to a first embodiment of the present invention will be explained in the following with reference to FIGS. 1 to 3.
As shown in FIG. 1, the brain wave examination apparatus 1 according to the present embodiment has a plurality of electrode apparatuses 2 for detecting brain waves which are disposed in contact with the scalp B of the subject A. Each electrode apparatus for detecting brain waves 2 is fixed in a hat- or helmet-shaped retaining member 4.

As shown in FIG. 2, the above-mentioned electrode apparatus for detecting brain waves 2 has an electrode apparatus main body 5, an electrode 6 provided exposed in the tip of the electrode apparatus main body 5, a cap 3 which is put over the tip of the electrode apparatus main body 5 in such a way as to cover the electrode 6, and a connection means 7 by which the cap 3 can be removably attached to the electrode apparatus main body 5.
The electrode apparatus main body 5 is formed in an approximately cylindrical shape and is fixed by insertion into through holes 4a provided in the retaining member 4.

The above-mentioned electrode 6 is a roughly disc-shaped plate and is fixed in the surface of the tip of the electrode apparatus main body 5. A cable 8 is connected to the electrode 6 through the interior of the electrode apparatus main body 5. In addition, the electrode 6 is made from metal or it is metal plated.
The cable 8 is connected to an electroencephalograph, not shown in the figure, and is such that the functional status of brain can be analyzed based on detected brain wave signals.

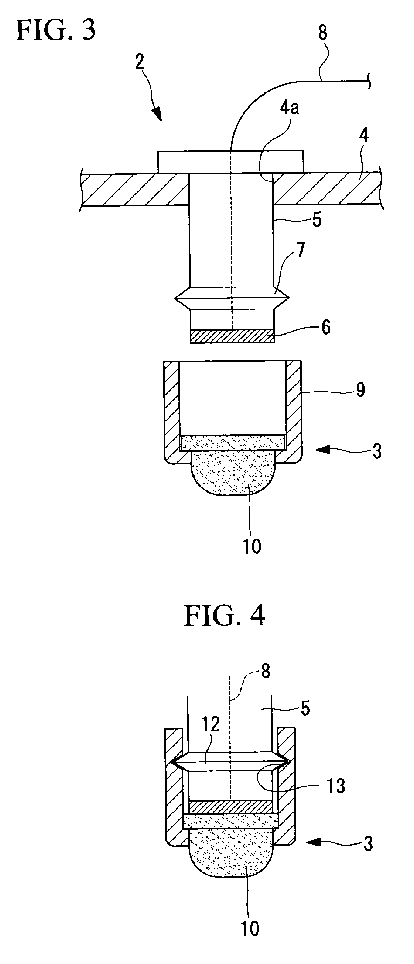

As shown in FIG. 3, the above-mentioned cap 3 comprises a roughly cylindrically-shaped cylindrical section 9 which covers the tip section of the above-mentioned electrode apparatus main body 5, and an elastic member 10 such as a sponge or a non-woven fabric supported in one end thereof. The elastic member 10 is formed such that, when the above-mentioned cap 3 is covering the electrode apparatus main body 5, it is disposed in close contact across the whole outer surface of the above-mentioned electrode 6, more specifically, it is formed having a contact surface with the electrode 6 which has a surface area greater than the surface area of electrode 6, and such that it is in close contact with the whole outer surface of the electrode 6 and contact resistant at the interface with the electrode is reduced as much as possible. The elastic member 10 is impregnated with an electrolyte 11 comprising a physiological saline solution or a mixture of a physiological saline solution and alcohol.

The above-mentioned connection means 7 is formed of an elastic supporting member (hereinafter also referred to as elastic supporting member 7) which has elasticity such as an O-ring or Y-packing. The elastic supporting member 7 is arranged, for example, completely encircling the outer peripheral surface of the electrode apparatus main body 5. When the cap 3 is put onto the electrode apparatus main body 5, a frictional force is generated between the elastic supporting member 7 of the outer peripheral surface of the electrode apparatus main body 5 and the inner surface of the cap 3, and it is possible for the cap to be maintained in an attached condition on the electrode apparatus main body 5.

By means of the brain wave examination apparatus 1 according to this embodiment constituted in this way, since the electrode apparatus for detecting brain waves 2 comprises an electrode apparatus main body 5 fixed in a retaining member 4 and a cap 3 which covers the tip of the electrode apparatus main body 5, it is possible to hygienically use the same brain wave examination apparatus 1 on a plurality of subjects A simply by replacing the caps 3. In addition, since the connection of the cap 3 and the electrode apparatus main body 5 is formed by the elastic supporting member 7 which maintains the two in an attached condition due to frictional force, the mounting and removal of the cap 3 from the electrode apparatus main body 5 is easy, and there is the advantage that there is no difficulty involved in the replacement operation. In addition, since the electrode comprises metal or metal plating, the conductivity is good and it is possible to prevent the occurrence of rust due to the electrolyte.

In addition, since the space between the electrode apparatus main body 5 and the cap is tightly sealed by the elastic supporting member 7, there is no risk of dust or dirt penetrating between the electrode 6 and the elastic member 10. In addition, simply by covering the electrode apparatus main body 5 with the cap 3, the elastic member 10 is brought into close contact with the entire electrode 6, and therefore, it is possible to reduce contact resistance and to detect the brain wave signal with high efficiency.

In addition, when the cap 3 has been installed on the electrode apparatus main body 5, since the elastic member 10 is disposed in the tip section of the electrode apparatus main body 5 which is in contact with the scalp B of the subject A, it is possible to ensure sufficient contact surface area with the scalp B by means of the elastic deformation of the elastic member 10. Furthermore, due to the elastic deformation of the elastic member 10, the electrolyte 11, which is soaked into the elastic member 10, flows out and wets the scalp, and it is possible to achieve an excellent conduction state between the electrode 6 and the scalp B.
As a result, by adopting a paste-less procedure, there are the benefits that it is possible to easily mount the brain wave examination apparatus and to reduce the burden on the subject A, and also that it is possible to detect stable brain wave signals without the use of paste.

In addition, the cap 3 which can be detached from the electrode apparatus main body 5 can be thrown away and replaced (as a disposable component) after each use, the cleaning of the retaining member 4 and electrode apparatus main body 5 after every use is unnecessary, and the labor and cost required in the maintenance of hygiene can be reduced.

Note that while the elastic supporting member 7 has been fixed to the outer peripheral surface of the electrode apparatus main body 5, it is also possible to fix it to the inner surface of the cap instead. The contact means is formed by an elastic supporting member 7 which is completely encircling, but it may also be formed by an elastic body such as rubber which is partially disposed in the circumferential direction.

In addition, in the present embodiment, as the connection means, an elastic support member 7 which maintains the connected condition between the cap 3 and the electrode apparatus main body 5 by frictional force has been adopted, but in place of this, as shown in FIG. 4, it also is possible to provide a projection 12, having a flange shape, in the outer peripheral surface of the electrode apparatus main body 5, and to provide a groove 13 on the inner surface of the cap 3 which engages the projection 12. By means of doing this, it is possible to maintain the connected condition with even greater certainty. For example, when the brain wave examination apparatus 1 is mounted on the head of a subject A, there are cases when the subject's A hair is pushed aside by the electrode apparatus for detecting brain waves, and even in such cases, it is possible to maintain the connected state of the cap 3 and the electrode apparatus main body 5.

Furthermore, it goes without saying that it is also possible for the groove to be formed in the outer peripheral surface of the electrode apparatus main body 5, and for the projection to be provided on the inner surface of the cap 3.
In addition, instead of the engagement of the projection 12 and the groove 13, it is also possible to achieve removable attachment by providing a mutually engageable male screw thread and female screw thread on the electrode apparatus main body 5 and the cap 3.

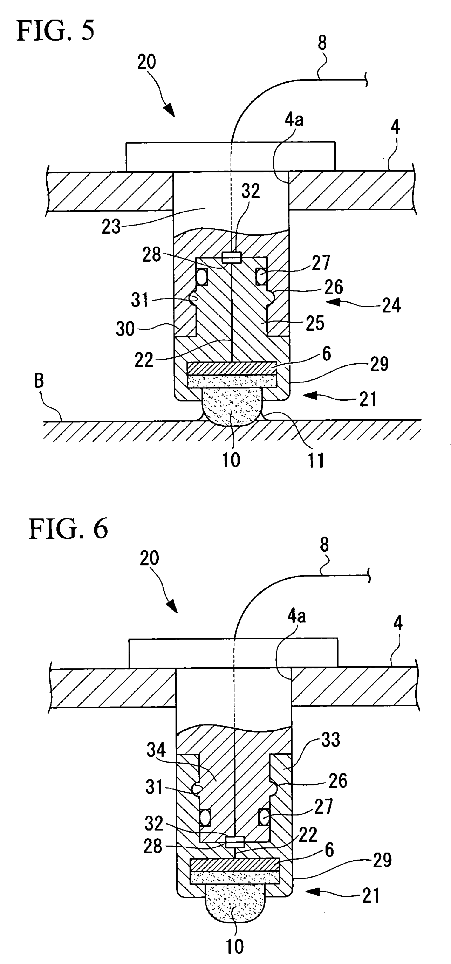

Next, an electrode apparatus for detecting brain waves 20 and a cap 21 according to a second embodiment of the present invention will be explained below with reference to FIG. 5.
The brain wave examination apparatus according to this embodiment differs from the brain wave examination apparatus 1 according the first embodiment in the electrode apparatus for detecting brain waves 20 and the cap 21. Note that in the explanation of the present embodiment, parts that are the same as in the brain wave examination apparatus 1, the electrode apparatus for detecting brain waves 2, and the cap 3 of the above-mentioned first embodiment have been given the same numbers and an explanation thereof has been omitted.

As shown in FIG. 5, the electrode apparatus for detecting brain waves 20 of the present embodiment comprises a cap 21, a disc-shaped electrode 6, an elastic member 10 which is disposed in close contact with the electrode 6, wiring 22 which is connected to the electrode 6, and a connection section 24 for connection to the electrode apparatus main body 23.
The electrode 6 and the elastic member 10 are the same as the electrode 6 and the elastic member 10 of the first embodiment. The connection section 24 comprises an engaging section 25 having a roughly cylindrical shape, a projection 26 provided on the outer peripheral surface of the engaging section 25, an O-ring-like seal member 27 which is provided on the outer peripheral surface in the same way, and a contact point (a second contact point) 28 which is formed by the exposure of one end of the above-mentioned wiring 22 at the surface of the tip of the engaging section 25.

The electrode 6 and the elastic member 10 are disposed in a condition of close contact with each other, and by means of a surrounding wall 29 which encloses their periphery, they are constituted such that the electrode 6 and the interface between the electrode 6 and the elastic member 10 are not exposed to the outside.

In addition, in the above-mentioned electrode apparatus main body 23, an engaging recess 30 into which the engaging section 25 of the cap 21 fits, a groove 31 provided in the inner peripheral surface of the engaging recess 30 and which engages the projection 26, and a contact point (a first contact point) 32 which is formed by the exposure of one end of the above-mentioned cable 8 at a position corresponding to the contact point 28 of the above-mentioned cap 21.

By means of an electrode apparatus for detecting brain waves 20 according to the present embodiment constituted in the above-described way, simply by the fitting together of the engaging section 25 of the cap 21 and the engaging recess 30 of the electrode apparatus main body 23, and the engagement of the projection 26 of the outer peripheral surface of the engaging section 25 with the groove 31 of the inner peripheral surface of the engaging recess 30, it is possible to maintain the cap 21 in an attached condition on the electrode apparatus main body 23. In addition, at that time, the first contact point 28 and the second contact point 32 are brought into contact, and the cable 8 and the electrode 6 are electrically connected. More specifically, the brain wave signals, obtained by brain wave detection carried out by bringing the elastic member 10 into contact with the head B of the subject A and wetting the scalp B with electrolyte 11, can be sent to an electroencephalograph, not shown in the figures, via wiring 22, first and second contact points 32 and 28, and the cable 8.

In addition, the seal member 27 provided in the engaging section 25 is squashed between the engaging section 25 and the inner peripheral surface of the engaging recess 30, and thereby the engaging surfaces of the engaging section 25 and the engaging recess 30 are circumferentially tightly sealed. Thereby, the contact section of the first contact point 28 and the second contact point 32 is disposed in a tightly sealed condition with respect to the exterior and the penetration of dust, dirt, and electrolyte 11 is prevented.

In this way, by means of the electrode apparatus for detecting brain waves 20 according to this embodiment, since the cap 21 which is provided with the electrode 6 can be replaceable attached, if the cap is taken as a disposable component, it is possible to achieve the same results as in the first embodiment of a reduction in the labor and cost involved in hygiene management. In addition, unlike the first embodiment, since it is possible to avoid the exposure of the electrode 6, it is possible to prevent the penetration of dust and dirt between the electrode 6 and the elastic member 10. In addition, since the contact section between the first contact point 32 and the second contact point 28 is protected in a tightly sealed condition by the seal member 27, it is possible to prevent the penetration of the dust, dirt, electrolyte 11, and the like, and to produce an excellent conductive condition.

Note that the projection 26, the groove 31, and the seal member 27 may be provided on either of the engaging section 25 or the engaging recess 30. In addition, while the cylindrically shaped engaging section 25 has been provided in the cap 21 and the engaging recess 30 has been provided in the electrode apparatus main body 23, it goes without saying that instead an engaging recess 33 may be provided in the cap 21 and an engaging section 34 may be provided in the electrode apparatus main body 23, as shown in FIG. 6.

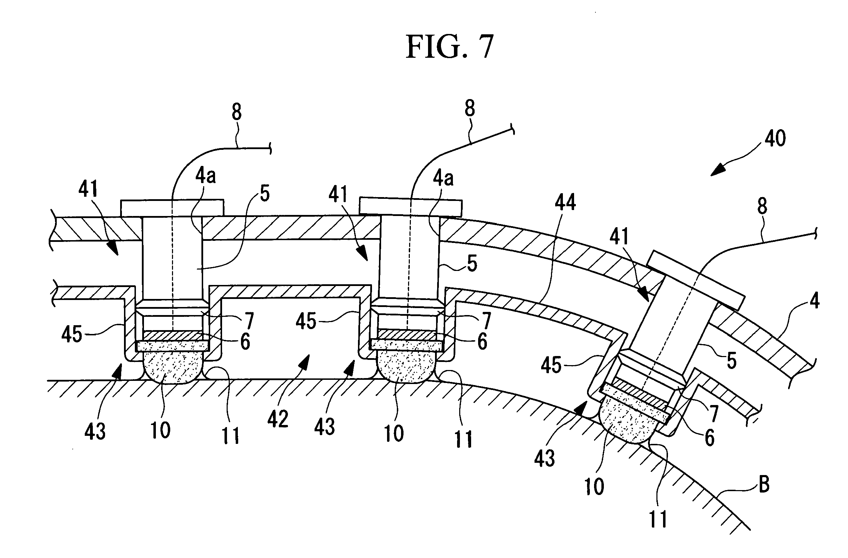

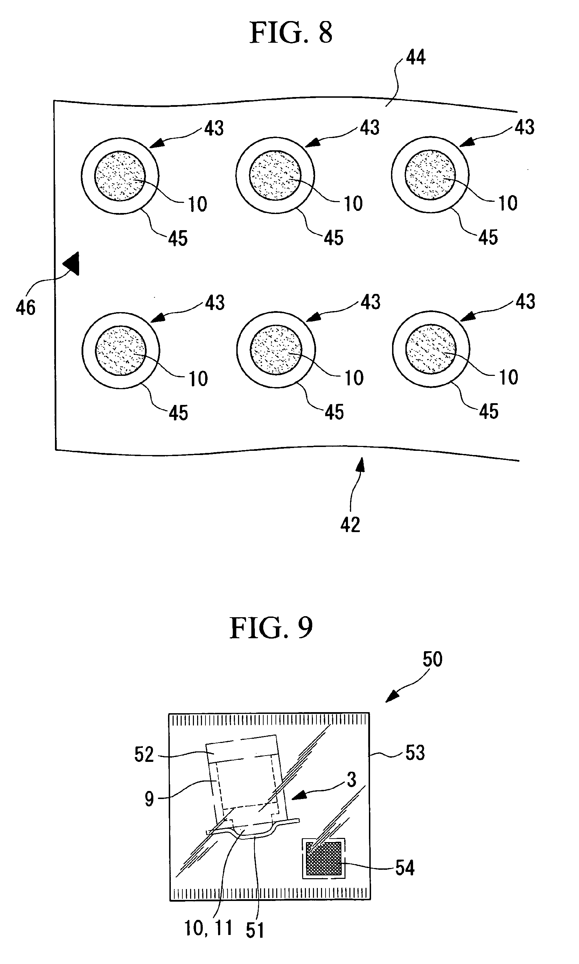

In the following, a brain wave examination apparatus 40, an electrode apparatus for detecting brain waves 41, and a cap 42 according to a third embodiment of the present invention will be explained with reference to FIGS. 7 and 8.
Note that in the explanation of the present embodiment, parts that are the same as in the brain wave examination apparatus 1, the electrode apparatus for detecting brain waves 2 and 20, and the cap 3 and 21 of the above-mentioned embodiments have been given the same references and an explanation thereof has been omitted.

The brain wave examination apparatus 40 and the electrode apparatus for detecting brain waves 41 according to the present embodiment differ from those of the first and second embodiments in the structure of the cap 42.
As shown in FIGS. 7 and 8, the cap 41 of the present embodiment comprises a plurality of cap sections 43 which cover a plurality of electrode apparatus main bodies 5 fixed in a retaining member 4, and also comprises a linking member 44 which links these cap sections 43. In the present embodiment, the electrode apparatus main body 5 and the cap sections 43 have substantially the same structure as the electrode apparatus main body 5 and the cap 3 in the first embodiment shown in FIG. 2 and FIG. 3.

In more detail, the cap sections 43 of the caps 41 of the present embodiment comprise a cylindrically shaped section 45 which covers an electrode 6 provided in the tip of each electrode apparatus main body 5, and an elastic member 10 which is supported in the tip of the cylindrically shaped section 45. The linking member 44 is a sheet-shaped member comprising a flexible or elastic member such as silicon rubber, for example, and links a plurality of the cap sections 43.
In addition, as shown in FIG. 8, a mark 46 (an instruction indicator) indicating the mounting direction thereof is provided on the linking member 44.

By means of a brain wave examination apparatus 40 of the present embodiment constituted in this way, by bending and flexing the linking member 44, the cap sections 43 can be mounted, as shown in FIG. 7, on all of the tip sections of the plurality of electrode apparatus main bodies 5 fixed in the retaining member 4, in such a way as to cover the electrodes 6 of the tips sections of the electrode apparatus main bodies 5. At this time, the cap 42 can be mounted using the mark 46 provided on the linking member 44 as a guide, for example, the mark 46 can be arranged in the center of the front of the retaining member 4. Thereby, it is possible to attach the cap 42 onto a plurality of electrode apparatus main bodies 5, collectively.

According to the brain wave examination apparatus 40 of the present embodiment, since the cap sections 43, which are mounted on a plurality of electrode apparatus main bodies 5, are united by the linking member 44, the number of disposable parts can be reduced, and management is easy. In addition, there is also the effect that it is possible to reduce the labor involved in mounting the disposable parts.
Note that in the explanation of this embodiment, as the structure of cap section 43, a structure identical to that of cap 3 of the first embodiment was adopted, but in place of this, the structures shown in FIG. 5 and FIG. 6 may be adopted for the electrode apparatus main body 5 and the cap 3.

In the following, a cap package 50 according to the first embodiment of the present invention will be explained with reference to FIG. 9.
The cap package 50 of the present embodiment comprises the cap 3 of the first embodiment, cover members 51 and 52 which are put on cap 3, and packing member 53 in which the cap 3 with cover members 51 and 52 fitted is packed in a tightly sealed condition. The elastic member 10 of cap 3 is soaked with an electrolyte 11.

The above-mentioned cover members 51 and 52 comprise a protective film 51 which covers the elastic member 10 of the cap 3 in an air-tight condition, and a protective part 52 which has a shape approximately the same as the tip section of the electrode apparatus main body 5 and which fits into the inside of the cylindrical part 9 of the cap 3. By means of the protective part 52 fitting inside the cap 3, the elastic member 10 which is exposed to the inside of the cap 3 is covered. By means of the protective film 51 and the protective part 52, the internally and externally exposed parts of the elastic member 10 are covered, and thereby, evaporation of the electrolyte 11 which is soaked into the elastic member 10 is inhibited, and there is no leakage to the outside. Furthermore, by removing the protective film 51 and the protective part 52 from the cap 3, the cap 3 is then in a condition to be mounted as it is on the tip section of the electrode apparatus main body 5 and used in the brain wave examination.

In addition, the above-mentioned packing member 53 is, for example, a transparent polyethylene pouch which is capable of sealing in an airtight state the cap with the cover members 51 and 52 attached, and is has a humidity sensor 54 on an inside surface thereof. The humidity sensor 54 is, for example, one which changes color in accordance with the humidity, and is attached to the inside surface in such a way that it is possible for that color change to be observed from the outside of the packing member 53.

By means of a cap package 50 formed in this way, since a humidity sensor 54 is disposed on the inside of the packing member 53, it is possible to check from the exterior of the packing member 53 whether or not the cap 3 which is stored in the packing member 53 is usable. In addition, at the time of use, the packing member 53 can be opened, the cap 3 can be taken out, the protective part 52 can be removed from the cap 3, and the elastic member 10 inside the cap 3 can be exposed, and thereby, mounting on the electrode apparatus main body 5 becomes possible. Next, by peeling off the protective film 51 after mounting the cap 3 on the electrode apparatus main body 5, it can be used as it is in brain wave examination. By doing this, since the elastic member 10 is covered by means of the protective film 51 up until just prior to use, it is possible to suppress the evaporation and the like of the electrolyte 11.

Consequently, by means of the cap package 50 of the present embodiment, when provided as a disposable part, it is possible to provide the cap 3, which is removably mountable on the tip of the electrode apparatus main body 5, in a state in which it can be used simply by mounting it onto the electrode apparatus main body 5 as it is and without the electrolyte 11, which is soaked into the elastic member, 10 drying out.

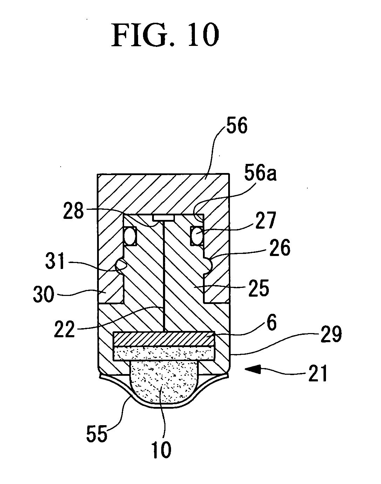

Moreover, the cap which is enclosed within the packing member 53 is not limited to the cap 3 of the first embodiment, and caps 21 and 42 shown in any of FIGS. 5 to 8 may be enclosed. FIG. 10 shows the situation in which a protective film 55 and a cover member 56 have been fitted for the case of a cap package 50 having the cap 21 shown in FIG. 5. Since the cap 21 of FIG. 10 has an electrode 6 and a contact point 28 connected to the electrode 6, by means of the seal member 27 arranged on the outer peripheral surface of the engaging section 25 sealing the inner surface of the engaging hole 56a of the protective part 56, it is possible to prevent the penetration of the electrolyte 11 to the contact point 28, even if by some chance the electrolyte 11 does leak.

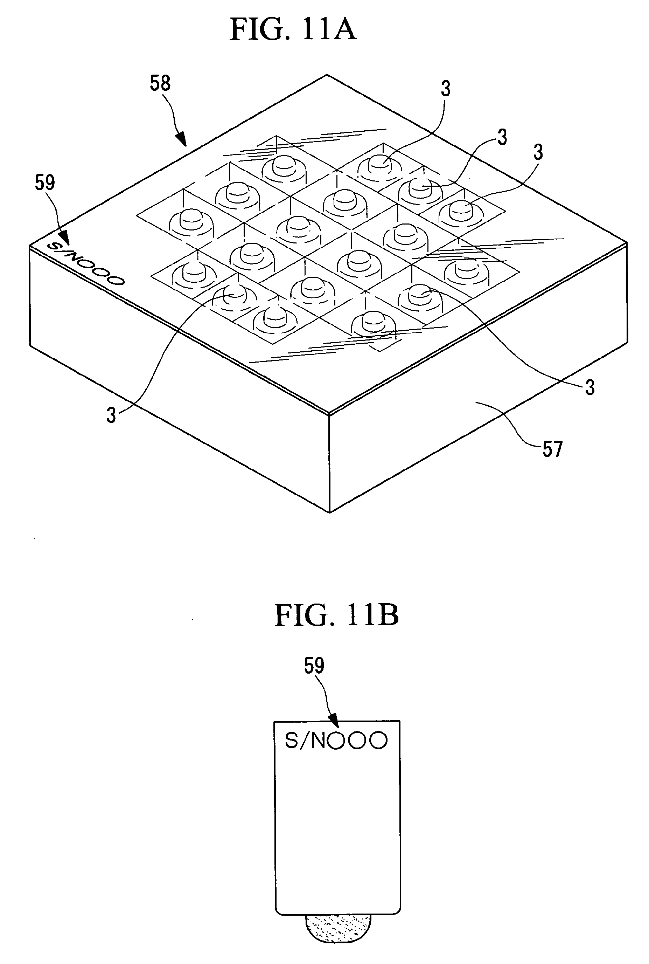

In addition, FIG. 11(a) shows an example of a cap package 58 having a packing member 57 which is capable of storing a plurality of caps 3 according to the first embodiment. The caps 3 are housed in a quantity for use at the same time. In FIG. 11(a), the housing sections for each cap 3 in the packing member 57 are provided corresponding to the arrangement of the electrode apparatuses for detecting brain waves 2 in the retaining member 4. In addition, the same identifying code 59, for example, a serial number, is printed on the outer surface of the packing member 57 and on the outer peripheral surface of the cap 3, as shown in FIG. 11(b).

By means of adopting this constitution, since the quantity of caps 3 required for a single brain wave detection are housed in the same packing member 57, after the replacement procedure, it is possible to check the caps 3 remaining in the packing member 57, and to prevent omissions in the replacement of the caps 3. In addition, since the caps 3 are housed corresponding to the arrangement of the electrode apparatuses for detecting brain waves 2 in the retaining member 4, it is possible to determine during the replacement of caps 3 which caps 3 have not yet been replaced. Furthermore, since caps 3 that have different serial numbers 59 are not supposed to used at the same time, by checking the serial number 59, it is possible to prevent the reuse of caps 3 on a plurality of subjects A.

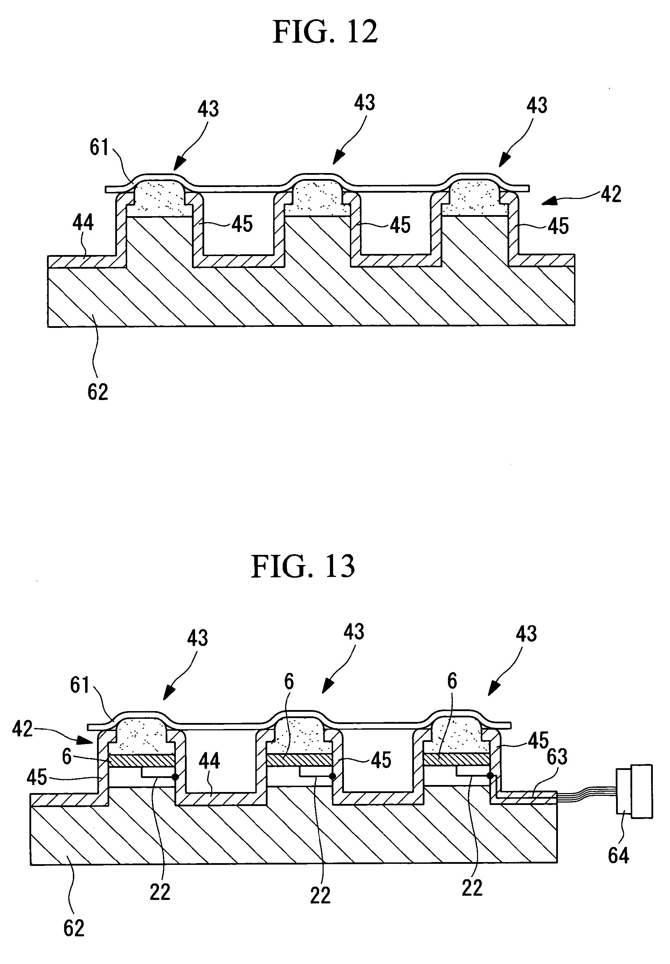

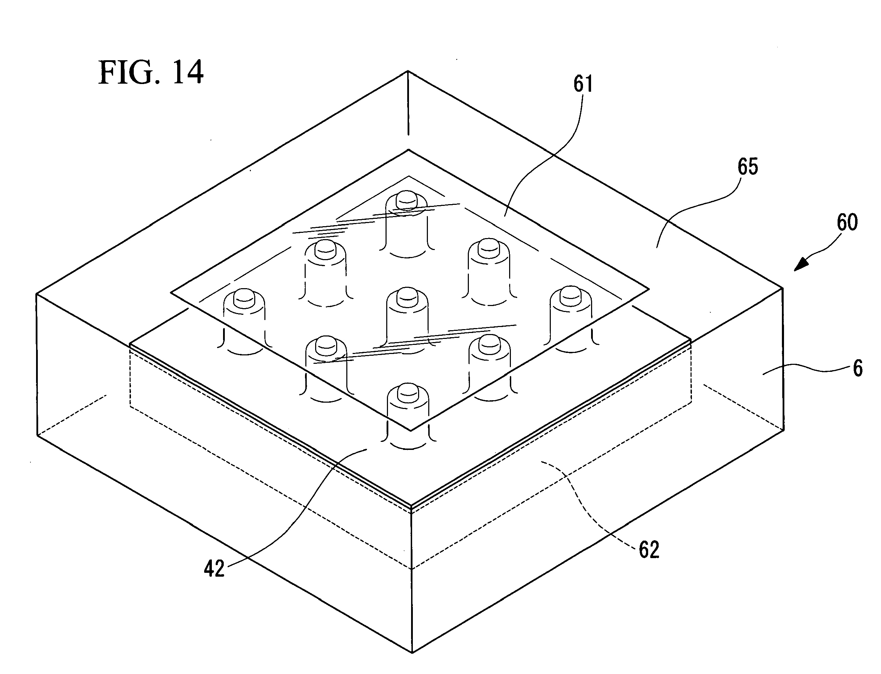

In addition, FIG. 12 shows a situation in which a protective film 61 and a protective part 62 have been fitted for the case of a cap package 60 having a cap 42 according to the third embodiment shown in FIG. 7. It is possible to use one protective film 61 and one protective part 62 with respect to a plurality of cap sections 43, and it is possible to reduce the number of parts. As cap section 43, it is possible to use one which does not have an electrode 6 as shown in FIG. 12, and it is also possible to use one which has an electrode 6 as shown in FIG. 13. In the figure, reference number 63 is a cable which connects to the wiring 22 within the cap, and it runs to the outside through the cap section 43 and the linking member 44 and is connected to connector 64.
In addition, as shown in FIG. 14, the packing member 65 may be in the form of a box capable of housing the entire cap 42 with the cover members 61 and 62 attached, as shown in FIGS. 12 and 13.

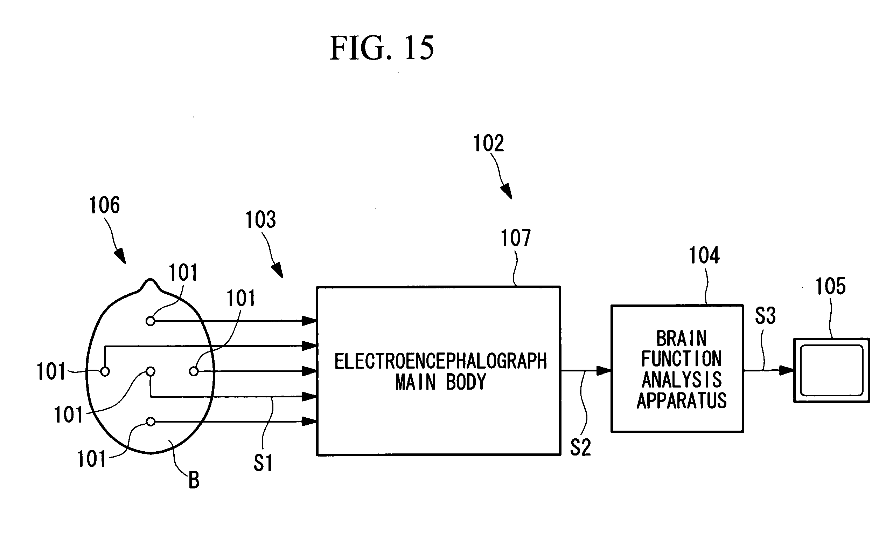

In the following, an electrode apparatus for detecting brain waves according to a fourth embodiment of the present invention will be explained with reference to FIGS. 15 to 18.
The electrode apparatus for detecting brain waves 101 (hereinafter referred to simply as the electrode apparatus 101) of this embodiment is used in the brain function examination system 102 shown in FIG. 15. The brain function examination system 102 comprises a brain wave detection apparatus 103 having a plurality of electrode apparatuses 101 disposed in contact with the scalp B, a brain function analysis apparatus 104, and a monitor 105 for displaying the analysis results.

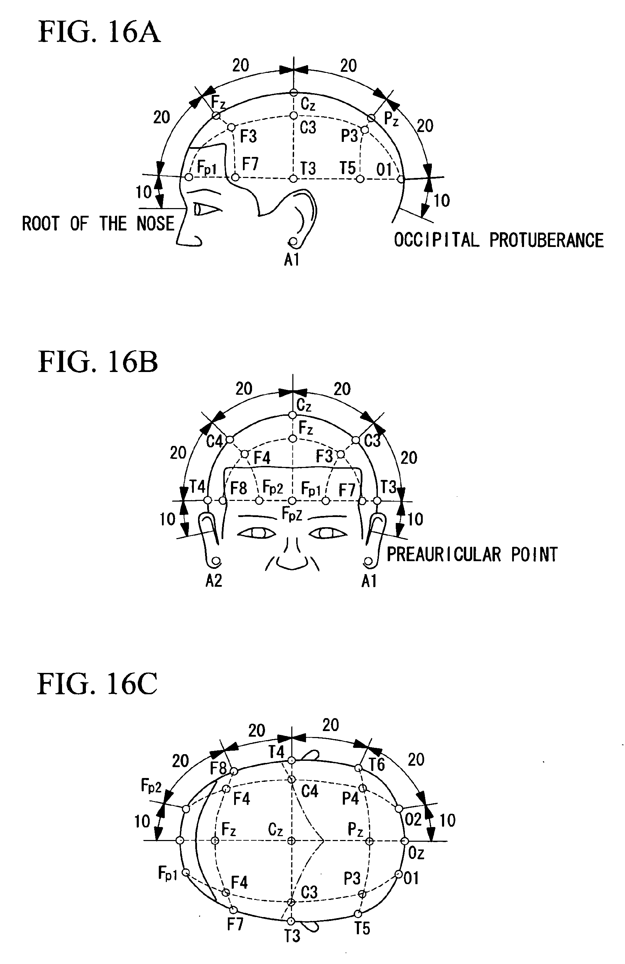

The brain wave detection apparatus 103 is an electroencephalograph and comprises a brain wave signal detector 106 comprising the plurality of electrode apparatuses 101 disposed in contact with the scalp B (FIG. 15 shows five electrode apparatuses 101 for simplicity of illustration, but normally there are twenty-one as shown in FIGS. 16(a), 16(b), and 16(c)), and an electroencephalograph main body 107 which outputs the electronic signal S1 detected by the electrode apparatus 101 as a brain wave signal S2.

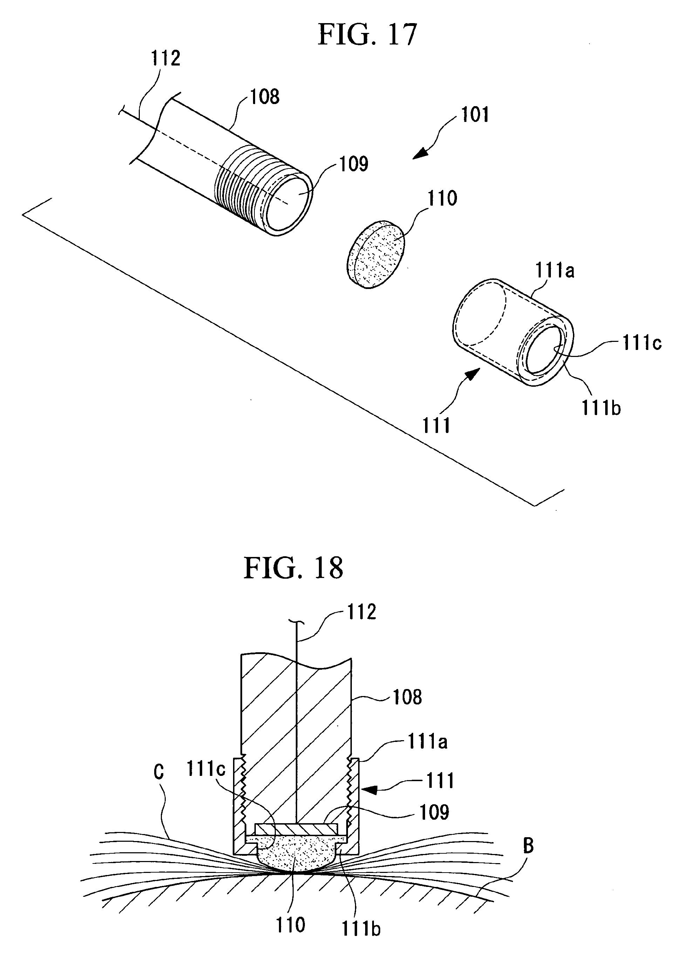

As shown in FIG. 2(a) to FIG. 2(c), with the brain wave signal detector 106, the plurality of electrode apparatuses 101 are disposed on the scalp at a predetermined ratio using standard positions as a standard. As shown in FIG. 17, the electrode apparatuses 101 comprises a metal electrode 109 disposed in the tip of the cylindrically shaped electrode apparatus main body 108, an elastic member 110 disposed so as to cover this metal electrode 109, and a cap 111 which is mounted on the electrode apparatus main body 108 in order to maintain the elastic member 110 in a condition covering the metal electrode 109.

The above-mentioned metal electrode 109 is, for example, a disc-shaped plate formed from metal or having a surface which is metal plated. Wiring 112 passes through the inside of the electrode apparatus main body 108 and is connected to the metal electrode 109, such that electronic signals S1 detected by the metal electrode 109 are transmitted to the electroencephalograph main body 107.
The above-mentioned elastic member 110 is, for example, an approximately disc-shaped sponge member and is soaked with an electrolyte. As the electrolyte, a physiological saline solution is preferable or a mixture of a physiological saline solution and alcohol is also suitable.

The above-mentioned cap 111 comprises a cylindrically shaped engaging section 111a which fits the outer surface of the tip of the above-mentioned electrode apparatus main body 108, and an inwardly directed flange-like section 111b which sandwiches the peripheral edge of the elastic member 110 against the metal electrode 109 disposed at the tip of the electrode apparatus main body 108. In addition, a circular opening 111c is formed to the inside of the inwardly directed flange-like section 111b.
FIG. 18 shows the case in which an electrode apparatus 101 has been brought into contact with a scalp B having hair C. In the figure, the electrode apparatus 101 is shown with the metal electrode 109, the elastic member 110, and the cap 111 assembled on the tip of the electrode apparatus main body 108. To the inside of the peripheral edge of the elastic member 110, which has been squashed between the inwardly directed flange-like section 111b of the cap 111 and the metal electrode 109, the elastic member 110 projects forward of the surface of the tip from the opening 111c of the cap 111.

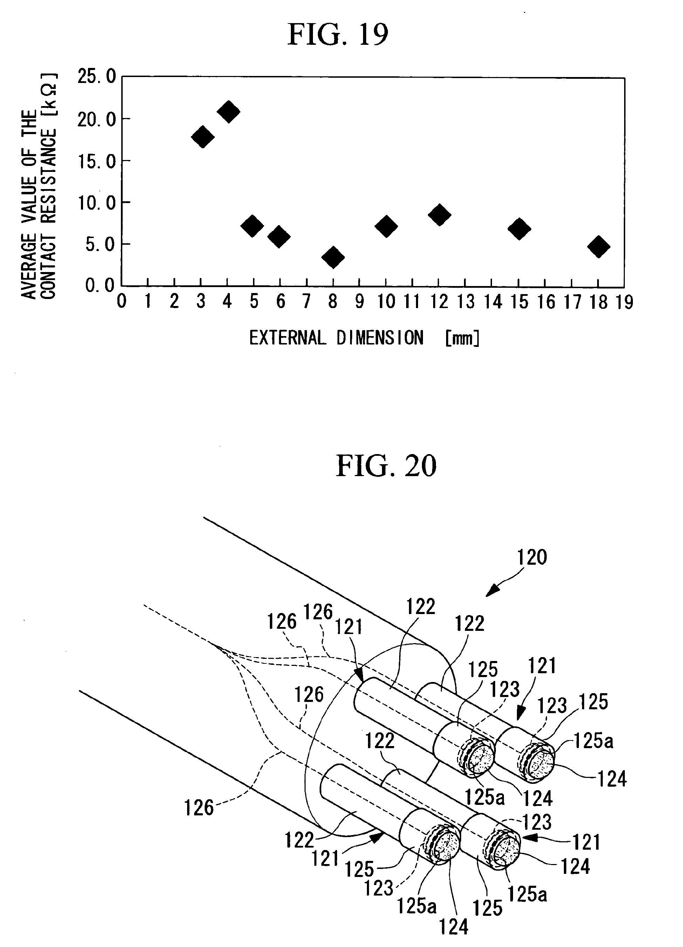

The area of this opening 111c is set to be about 19 mm² or greater.
Table 1 and FIG. 19 show, for the selection of the above-mentioned area, the measurement results of contact resistance values for each of a plurality of electrode apparatuses 101 disposed in contact with the scalp B. The opening 111c was circular, the size of the diameter of the opening 111c was varied, and the contact resistance was measured. Table 1 shows the contact resistance value for each measurement point and the average values therefor. FIG. 19 is a graph showing the relationship between the size of the diameter of the opening section 111c and the averaged value of the contact resistance. According to these measurement results, when the diameter of the opening 111c is less than 5 mm, the contact resistance is large, and when the diameter is 5 mm or greater, in other words, when the area of the opening 111c is approximately 19 mm² or greater, the average value for the contact resistance is 10KΩ or less.

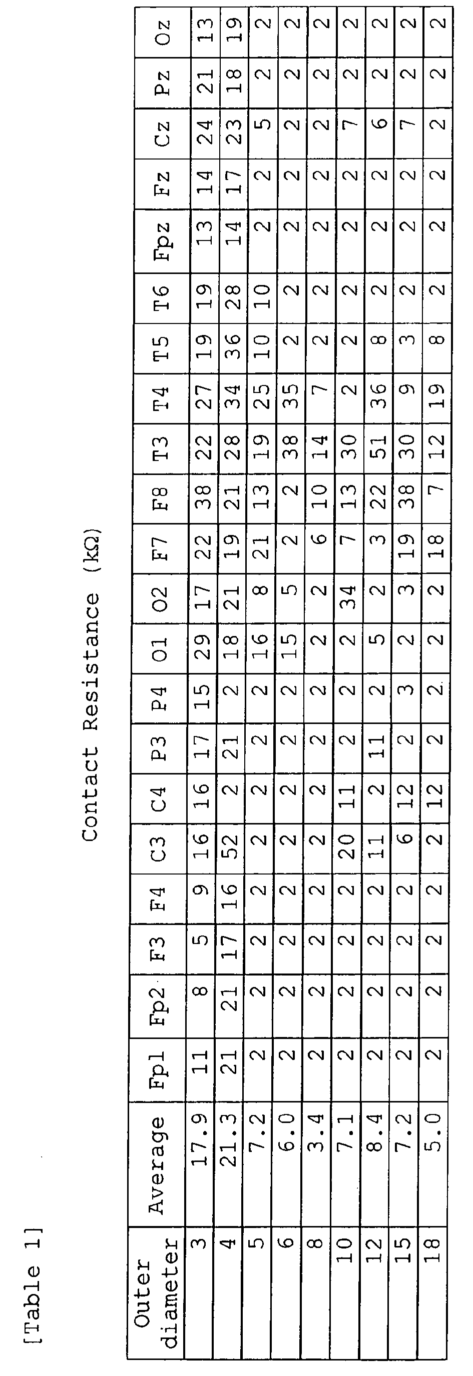

Consequently, by making the area of the opening approximately 19 mm² or greater, it is possible to keep the contact resistance low. On the other hand, as shown in Table 1 and FIG. 19, if the diameter of the opening 111c is 5 mm or greater, there is not much variation in the contact resistance value. Consequently, it is sufficient for the area of the opening 111c to be 19 mm² or greater, and there is no need to make it any larger. Since a plurality of electrode apparatuses 101 are disposed in contact with the scalp B, it is necessary to ensure sufficient spacing from other neighboring electrode apparatuses 101. In consideration of problems such as mechanical interference and electrical short circuits, it is preferable for the diameter of the opening 111c to be 20 mm or less, in other words, it is preferable for the area of the opening 111c to be 314 mm² or less.
In addition, the brain function analysis apparatus 104 is structured such that the brain wave signal S2 output from the electroencephalograph main body 107 is input to the brain function analysis apparatus 104, the state of brain function is judged based on the brain wave signal S2, and a judgment signal S3 is output.

By means of an electrode apparatus 101 of the present embodiment constituted in the above-described way, since the area of the opening 111c is set to be 19 mm² or greater, even if there is hair C present, it is possible to maintain a sufficiently low contact resistance with the scalp C, and by obtaining a stable brain wave signal S2, it is possible to analyze and display functional status of brain with good accuracy. In addition, by setting the area of the opening 111c to 314 mm² or less, it is possible to avoid mechanical and electrical interference with other neighboring electrode apparatuses 101, and thereby, it is possible to perform the judgment of functional status of brain with even greater accuracy.

In addition, in the electrode apparatus 101 of the present embodiment, since the metal electrode 109 is made from metal or is metal plated, there are the effects that it is possible to prevent increases in resistance in the metal electrode 109, and furthermore, that due to the anti-corrosive effects of metal, it is possible to prevent increases in resistance which accompany use before they occur.

In addition, by means of forming the electrolyte from physiological saline solution, it is possible to prevent increases in contact resistance. Furthermore, by the electrolyte comprising a mixture of physiological saline solution and alcohol, there are the effects that it is possible to increase the cleaning effect due to the alcohol with regard to sebum in the scalp, and to prevent increases in contact resistance due to the sebum.
Note that this explanation has been made giving, as an example, a sponge material capable of being impregnated with the electrolyte, but instead, a non-woven fabric may be used. In addition, the elastic member 110 and the cap 111 may be unitarily fixed.

In the following, an electrode apparatus according to a fifth embodiment of the present invention will be explained with reference to FIG. 20.
As shown in FIG. 20, the electrode apparatus 120 of the present embodiment comprises a plurality of tip sections 121 which are arranged mutually separated from each other. Each tip section 121 comprises, in the same way as in the fourth embodiment, a cylindrical electrode main body 122, a metal electrode 123 disposed in the tip of the electrode main body 122, an elastic member 124, and a cap 125. Wirings 126 which are connected to a plurality of metal electrodes 123 are all interconnected.
In addition, the total area of the openings 125a of the plurality of caps 125, in other words, the total area of the elastic members 124 which project forward from the openings 125a, is 19 mm² or greater.

By means of an electrode apparatus 120 according to the present embodiment having the above-described constitution, the total surface area of the elastic member 124 which is impregnated with the electrolyte is 19 mm² or greater, it is possible to achieve sufficiently low contact resistance values, in the same way as in the fourth embodiment.
In addition, by means of the electrode apparatus 120 of the present embodiment, by disposing a plurality of tip sections 121 separated at intervals, when the tip sections 121 are in contact with the scalp B, the hair C is positioned in the intervals between the tip sections 121, and it is easy to bring the tip sections 121 into contact with the scalp B which has been exposed from between the hair C which has been pushed aside. In other words, with the electrode apparatus 120 of the present embodiment, since the plurality of tip sections 121 functions like the teeth of a comb and pushes the hair C aside, there is the effect that it is possible to achieve stable and lower contact resistance values through more reliable contact with the scalp.

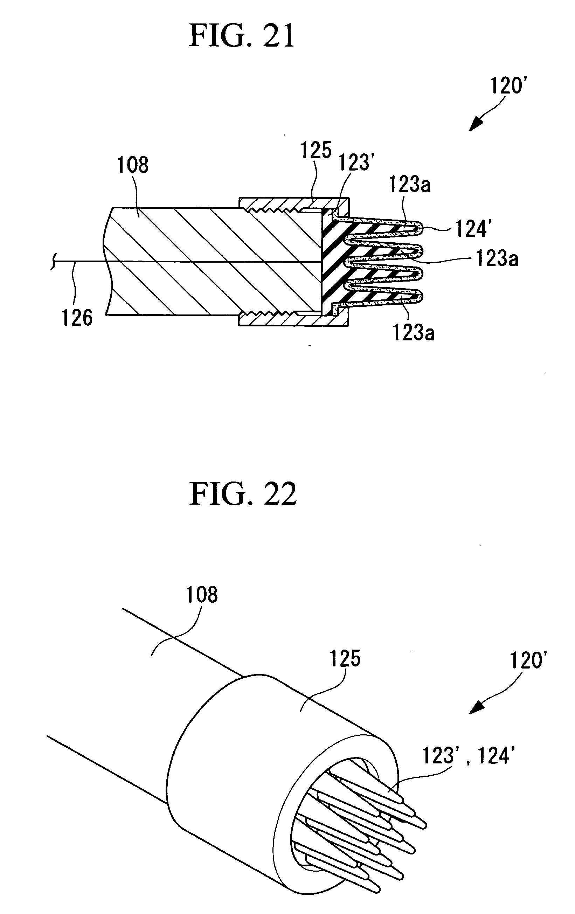

Note that in place of the electrode apparatus 120 having the plurality of metal electrodes 123, elastic members 124, and caps 125 as in the above-described embodiment, as shown in FIG. 21 and FIG. 22, the metal electrode 123' itself can be made to have a plurality of pointed tip sections 123a, and as the elastic member 124', one which is formed to cover all of the tip sections 123a may be used. According to the electrode apparatus 120' constituted in this way, the pointed tip sections 123a of the metal electrode 123' are inserted into a unitary elastic member 124', and the elastic member 124' presses against the metal electrode 123' and is held due to the elasticity of the elastic member 124'. In addition, since the plurality of divided tip sections 123a functions like the teeth of a comb and push the hair aside, it is possible to achieve stable and lower contact resistance values through more reliable contact between the metal electrode 123' and the scalp B.

In the following, an electrode apparatus 130 according to a sixth embodiment of the present invention will be explained with reference to FIG. 21.
As shown in FIG. 21, in the electrode apparatus 130 according to the present embodiment, the metal electrode 131 is formed having spherical surface of radius r, and the elastic member 132 which covers the metal electrode 131 is also formed having a spherical shape following the shape of the metal electrode 131. The electrode main body 133, the cap 134, and the wiring 135 are the same as in the fourth and fifth embodiments.

As shown in FIG. 21, by means of a constitution such as this, when it is not possible to dispose the electrode 130 at right angles with respect to the scalp B due to the shape of the head, it is possible to bring the spherically shaped elastic member 132 into close contact with the scalp B even at places at which the electrode is disposed at angle of inclination θ. By making the radius of curvature r large, the contact area between the scalp B and the elastic member 132 is increased, and it is possible for it to be formed so that the contact area is 19 mm² or greater, and it is possible to reduce contact resistance in the same way as in the fourth and fifth embodiments.

## Claims

1. An electrode apparatus for detecting brain waves which is disposed in contact with the scalp and detects brain wave signals, comprising:
a rod-shaped electrode apparatus main body having an electrode disposed in a tip thereof;
a cap which is mountable on the tip of the electrode apparatus main body and which has an elastic member containing an electrolyte and arranged in close contact so as to cover the electrode; and
a connection means for connecting the cap in a detachable manner to the electrode apparatus main body.

2. An electrode apparatus for detecting brain waves which is arranged in contact with the scalp and detects brain wave signals, comprising:
a rod-shaped electrode apparatus main body having a first contact point disposed in a tip thereof;
a cap mounted such that the tip of the electrode apparatus main body is covered and having a second contact point for contact with the first contact point; and
a connection means for connecting the cap in a detachable manner to the electrode apparatus main body;
the cap having an electrode for connection to the second contact point and an elastic member containing an electrolyte and disposed in close contact so as to cover the electrode.

3. An electrode apparatus for detecting brain waves according to claim 2, wherein the connection means comprises a seal member which maintains a contact section of the first contact point and the second contact point in a watertight condition when the cap is mounted on the electrode apparatus main body.

4. The apparatus for detecting brain waves according to claim 1 or claim 2, wherein a plurality of the caps are integrally connected by means of a linking section.

5. The apparatus for detecting brain waves according to claim 4, wherein the linking section comprises a flexible material.

6. The cap according to claim 4, wherein an indicator mark for indicating a mounting direction is provided on the linking section.

7. The electrode apparatus for detecting brain waves according to claim 1 or claim 2, wherein the connection means comprises an elastic supporting member which is fixed to either one of the cap and the electrode apparatus main body in an engaging section of the cap and the electrode apparatus main body, and which maintains a connected condition by means of frictional force with the other one of the of the cap or the electrode apparatus main body.

8. The electrode apparatus for detecting brain waves according to claim 7, wherein the elastic supporting member comprises an O-ring or Y-packing.

9. The electrode apparatus for detecting brain waves according to claim 1 or claim 2, wherein the connection means comprises a female screw thread provided on one of the cap and the electrode apparatus main body, and a male screw thread provided on the other one of the cap and the electrode apparatus main body and which engages the female screw thread.

10. The electrode apparatus for detecting brain waves according to claim 1 or claim 2, wherein the connection means comprises a projection which projects in a direction orthogonal to a connection direction and provided on one of the cap and the electrode apparatus main body, and a groove for receiving the projection when in a connected condition which is provided on the other one of the cap and the electrode apparatus main body.

11. The electrode apparatus for detecting brain waves according to claim 1 or claim 2, wherein an area of a surface of a tip of the cap which makes contact with the scalp is not less than approximately 19 mm² and not greater than approximately 314 mm².

12. The electrode apparatus for detecting brain waves according to claim 1 or claim 2, wherein the electrode comprises metal or is metal plated.

13. The electrode apparatus for detecting brain waves according to claim 1 or claim 2, wherein the electrolyte comprises physiological saline solution.

14. The electrode apparatus for detecting brain waves according to claim 1 or claim 2, wherein the electrolyte comprises a mixture of physiological saline solution and alcohol.

15. The electrode apparatus for detecting brain waves according to claim 1 or claim 2, wherein the electrode apparatus main body comprises a plurality of electrodes, and a total area of surfaces of tips of the caps which are disposed making close contact so as to cover all the electrodes is not less than approximately 19 mm² and not greater than approximately 314 mm².

16. The electrode apparatus for detecting brain waves according to claim 1 or claim 2, wherein an electrode having an approximately comb tooth-like shape is provided in the electrode apparatus main body, and a total area of a surface of a tip of the cap which is disposed making close contact so as to cover the electrode is not less than approximately 19 mm² and not greater than approximately 314 mm².

17. The electrode apparatus for detecting brain waves according to claim 1 or claim 2, wherein a tip section of the electrode is formed having a roughly spherical surface, and a surface of a tip of the cap is formed having a roughly spherical surface shape following the spherical surface of the electrode.

18. A package comprising a cap according to any one of claims 1 to 17 and a packing member for housing the cap in an airtight state.

19. A package according to claim 18 further comprising a cover member for covering a surface of the elastic member in the cap.

20. A package according to claim 18, wherein a humidity sensor is disposed within the packing member which is visible from the outside.

21. A package according to claim 18, wherein the packing member houses a plurality of caps.

22. A package according to claim 21 wherein the plurality of caps are housed in the packing member corresponding to the arrangement of the caps at the time of the brain wave detection.

23. A package according to claims 21 wherein an identical identifying code is provided on the cap and the packing member.
